# EUROPEAN PATENT APPLICATION

(11) **EP 4 349 445 A1**
(43) Date of publication of application: **10.04.2024**
(21) Application number: 22816119.6
(22) Date of filing: 31.05.2022
(51) Int. Cl.: B01D 9/02, C07C 51/43, C07C 57/055

(54) **TANK USED IN REFINING DEVICE**

(30) Priority: 02.06.2021 JP 2021093032
(71) Applicant: Nippon Shokubai Co., Ltd., Osaka-shi, Osaka 541-0043 (JP)
(72) Inventor: FUKUMOTO, Toshiya, Himeji-shi, Hyogo 671-1282 (JP); TAKEMOTO, Yasutaka, Himeji-shi, Hyogo 671-1282 (JP); MUKAE, Masashi, Himeji-shi, Hyogo 671-1282 (JP)
(74) Representative: Schön, Christoph
(86) International application number: PCT/JP2022/022166
(87) International publication number: WO 2022/255373

(57) **Abstract**

Provided is a method for stably obtaining a product. The present invention relate to a tank for use in a purification apparatus, the tank being: at least one of a crystallization tank that forms a slurry containing crystals of a compound or a ripening tank that is capable of keeping crystals of a compound suspended therein, the tank including a nozzle for feeding a compound-containing solution or a slurry containing crystals of a compound into the tank while the solution or slurry is brought into contact with an inner wall surface of the tank; and a heating mechanism for heating an area of the inner wall surface with which the compound-containing solution or the slurry containing crystals of a compound is to be brought into contact.

## Description

### TECHNICAL FIELD

The present invention relates to a tank for use in purification apparatus. Specifically, the present invention relates to a tank for use in a purification apparatus, a purification apparatus, a method for producing a compound, and a method for purifying a compound.

### BACKGROUND ART

Purification apparatuses have been widely used industrially to purify compounds that are used as raw materials for resins, for example. In many fields of the chemical industry, high-quality compounds with reduced impurities have been required, and for such compounds, various studies have been conducted on better purification apparatuses.

Industrially, many of crude compounds, which are compounds before purification, are purified through continuous purification processes. Disclosed is a method for producing acrylic acid including: collecting and crystallization purifying a gas containing acrylic acid obtained by catalytic vapor phase oxidation of a raw material gas; and returning the decomposed product of the Michael adducts of acrylic acid contained in a residual mother liquor to the collecting step, for example (see, for example, Patent Literature 1).

In order to obtain a high-purity compound in a higher yield, the purification uses a tank that forms a slurry containing crystals of a compound (crystallization tank) and/or a tank that grows crystals of a compound (ripening tank). Patent Literatures 2 to 4 disclose conventional purification methods using a crystallization tank and/or a ripening tank.

### CITATION LIST

### - Patent Literature

Patent Literature 1: JP 2007-182437 A
Patent Literature 2: JP 2005-28214 A
Patent Literature 3: JP 2012-140471 A
Patent Literature 4: JP 2002-204937 A

### SUMMARY OF INVENTION

### - Technical Problem

As described above, better purification apparatuses for producing compounds have been desired, and methods for stably obtaining products (compounds) have been desired. The present invention has been made in view of the above-mentioned current state of the art, and aims to provide a method for stably obtaining a product.

### - Solution to Problem

The present inventors have studied a method for stably obtaining a product, and focused on a tank for use in a purification apparatus. The present inventors have encountered the following problem. When a solution or slurry is fed to a crystallization tank that forms a slurry containing crystals of a compound or a ripening tank that is capable of keeping crystals of a compound suspended therein while the solution or slurry is fed along (flowing down) the inner wall surface of the tank in order to prevent liquid splashing, fine crystals formed from the solution or a mother liquor in the slurry adhere to the inner wall surface and the fine crystals as crystal nuclei grow into coarse crystals. The present inventors have extensively studied and found that such a problem can be solved by a tank including a nozzle for feeding a solution or slurry while the solution or slurry is brought into contact with an inner wall surface of the tank and a heating mechanism for heating an area of the inner wall surface with which the solution or slurry is to be brought into contact. Specifically, when the solution or slurry is fed to such a tank, liquid splashing is prevented, fine crystals are prevented from adhering to the inner wall surface of the tank, and the fine crystals as nuclei are prevented from growing. Thereby, a product can be stably obtained, and the present invention has been completed.

That is, the present invention relates to a tank for use in a purification apparatus, the tank being at least one of a crystallization tank that forms a slurry containing crystals of a compound or a ripening tank that is capable of keeping crystals of a compound suspended therein, the tank including a nozzle for feeding a compound-containing solution or a slurry containing crystals of a compound into the tank while the solution or slurry is brought into contact with an inner wall surface of the tank; and a heating mechanism for heating an area of the inner wall surface with which the compound-containing solution or the slurry containing crystals of a compound is to be brought into contact.

### - Advantageous Effects of Invention

The purification apparatus of the present invention can stably provide a product.

### BRIEF DESCRIPTION OF DRAWINGS

FIG. 1 is a schematic cross-sectional side view of an exemplary tank of the present invention.
FIG. 2 is a perspective view of a nozzle and a heating mechanism in the tank shown in FIG. 1.
FIG. 3 is a schematic cross-sectional side view of another exemplary tank of the present invention.
FIGS. 4(a) to 4(c) are schematic views of exemplary nozzles used in the tank of the present invention.

### DESCRIPTION OF EMBODIMENTS

The present invention will be described in detail below.

It should be noted that combinations of two or more of the preferred features of the present invention described below are also preferred embodiments of the present invention.

The following first describes a tank of the present invention (a crystallization tank and/or a ripening tank for use in a purification apparatus), followed by descriptions of a purification apparatus of the present invention, a method for producing a compound of the present invention, and a method of purifying a compound of the present invention.

### (Tank for use in purification apparatus)

The tank is a crystallization tank that forms a slurry containing crystals of a compound and/or a ripening tank that is capable of keeping crystals of a compound suspended therein.

The tank is provided with a nozzle for feeding a compound-containing solution or a slurry containing crystals of a compound (herein also simply referred to as a solution or slurry) into the tank while the solution or slurry is brought into contact with an inner wall surface of the tank and a heating mechanism for heating an area of the inner wall surface with which the compound-containing solution or the slurry containing crystals of a compound is to be brought into contact.

The nozzle is to feed a compound-containing solution or a slurry containing crystals of a compound into the tank while the solution or slurry is brought into contact with an inner wall surface of the tank. The nozzle typically extends from the outside to the inside of the tank, and the tip (a feed port that feeds the solution or slurry into the tank) of the nozzle in the tank is directed to and close to the inner wall surface of the tank.

The nozzle may penetrate the top roof of the tank, for example. Here, the nozzle may vertically penetrate the top roof of the tank and have a bend in the tank, and the tip of the nozzle may be directed to the inner wall surface, or the nozzle may obliquely penetrate the top roof of the tank. Preferably, the nozzle vertically penetrates the top roof of the tank and has a bend in the tank, and the tip of the nozzle is directed to the inner wall surface.

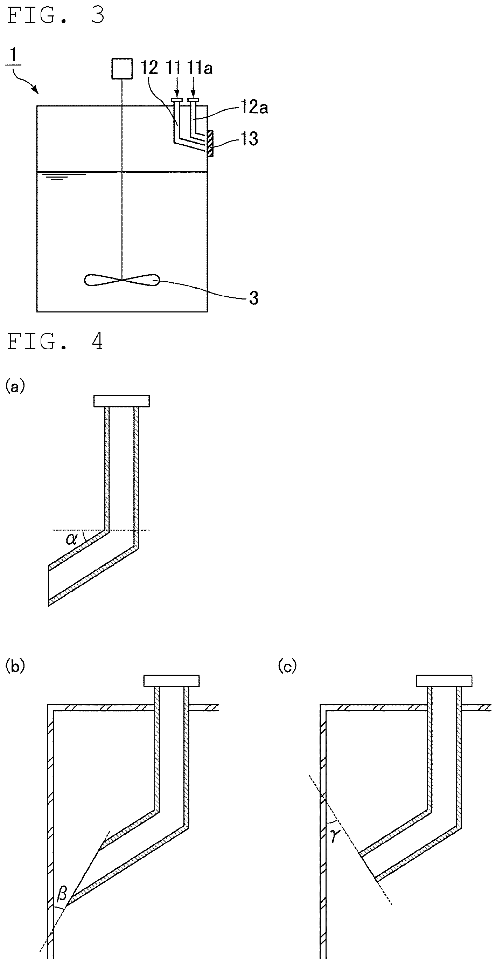

For example, the tip of the nozzle is preferably directed downward. In particular, to stably obtain a product, the tip is more preferably directed downward at an angle of 5° or more, more preferably at an angle of 10° or more, still more preferably at an angle of 15° or more, with respect to the horizontal direction. To feed the solution or slurry while the solution or slurry is brought into contact with and along the inner wall surface more suitably, the tip of the nozzle is preferably directed downward at an angle of 70° or less, still more preferably 60° or less, particularly preferably 50° or less, with respect to the horizontal direction. The angle at which the tip of the nozzle is directed downward with respect to the horizontal direction corresponds to the angle α between the horizontal direction indicated by the broken line and the tip of the nozzle in FIG. 4(a).

The distance (shortest distance) between the tip of the nozzle and the inner wall surface is preferably 0.1 to 5 times, more preferably 0.2 to 2 times, still more preferably 0.3 to 1 time, particularly preferably 0.4 to 0.9 times, most preferably 0.6 to 0.7 times, of the inner diameter of the nozzle.

The inner diameter of the nozzle is preferably 2 to 300 mm, more preferably 10 to 200 mm.

The nozzle may be made of any material and is preferably made of a metal or an alloy.

The shape of the tip of the nozzle is not limited, and may be flat or sharp. The tip face (constituted by an opening and a peripheral portion thereof) of the nozzle preferably forms with the inner wall surface an angle of 15° or less, more preferably 10° or less, still more preferably 5° or less. Particularly preferably, the tip face is parallel to the inner wall surface. The angle formed by the tip face of the nozzle and the inner wall surface corresponds to the angle β in FIG. 4(b) or the angle γ in FIG. 4(c).

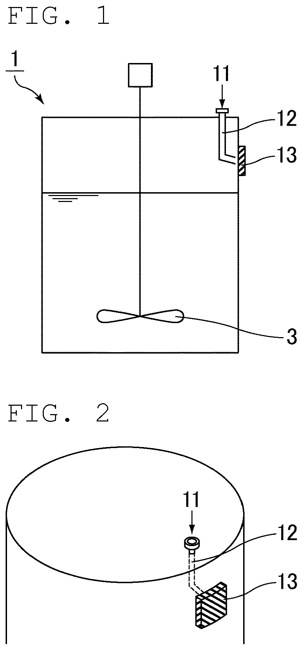

Although FIG. 1, which is shown below, shows a tank 1 including one nozzle that sends a solution or slurry 11 to the tank 1 and one feed port for the solution or slurry, the tank 1 may include multiple nozzles as shown in FIG. 3. In this case, at least one of the nozzles should satisfy the above-mentioned structure of the present invention. Preferably, all of the nozzles satisfy the structure of the present invention. One or more heating mechanisms may be provided for a group of two or more nozzles, or one or more heating mechanisms may be provided for each nozzle.

The heating mechanism is to heat an area of the inner wall surface with which the compound-containing solution or the slurry containing crystals of a compound is to be brought into contact.

Non-limiting examples of the heating mechanism include a heating medium, a steam trace system, an electric trace system, and known heaters for adjusting the environmental temperature of the tank. The area of the inner wall surface with which the compound-containing solution or the slurry containing crystals of a compound is to be brought into contact may serve as a heating mechanism.

In particular, in the tank of the present invention, the heating mechanism is preferably a jacket-type heating mechanism.

When the heating mechanism is a jacket-type heating mechanism, the jacket may be made of any material such as a metal (e.g., stainless steel or carbon steel) or a resin.

Preferably, the jacket-type heating mechanism on a wall surface of the tank has a vertical length and a horizontal length, each of which is equal to or greater than an inner diameter of the nozzle.

The vertical length is more preferably twice or more, still more preferably three times or more, particularly preferably five times or more the inner diameter of the nozzle.

The upper limit of the vertical length to the inner diameter of the nozzle is not limited, and for example, the vertical length is 20 times or less the inner diameter of the nozzle.

The horizontal length is more preferably twice or more, still more preferably three times or more, further more preferably four times or more, particularly preferably five times or more the inner diameter of the nozzle.

The upper limit of the horizontal length to the inner diameter of the nozzle is not limited, and for example, the horizontal length is 100 times or less the inner diameter of the nozzle.

The jacket-type heating mechanism may have a rectangular shape or another shape (e.g., a circular shape, a polygonal shape) and preferably has a rectangular shape.

The tip of the nozzle is preferably located such that if the centerline of the opening of the tip of the nozzle is extended, the centerline reaches a region in the heating mechanism at or above 1/6, more preferably a region at or above 1/5, still more preferably a region at or above 2/5 vertically from the bottom of the heating mechanism.

The tip of the nozzle is preferably located such that if the centerline of the opening of the tip of the nozzle is extended, the centerline reaches a region in the heating mechanism at or below 9/10, more preferably a region at or below 4/5 vertically from the bottom of the heating mechanism.

The tip of the nozzle is preferably located such that if the centerline of the opening of the tip of the nozzle is extended, the centerline reaches a central region in the heating mechanism between 1/10 horizontally from the left side of the heating mechanism and 1/10 horizontally from the right side of the heating mechanism. More preferably, the extended line reaches a region in the heating mechanism between 1/5 horizontally from the left side of the heating mechanism and 1/5 horizontally from the right side of the heating mechanism.

The jacket may be composed of divided sections, and the sections may be operated at different temperatures (with different heating mediums).

The outside of the jacket may be provided with a heat insulating material, a tracing system, and the like.

The inside of the jacket may be provided with any structure such as a structure that promotes heat transfer, such as a baffle.

The jacket preferably has an average thickness (the width of the space where the heating medium flows) of 5 to 200 mm, for example.

The heat flux from the jacket to the wall surface of the tank is preferably 100 W/m² or more, more preferably 200 W/m² or more, still more preferably 500 W/m² or more.

The upper limit of the heat flux from the jacket to the wall surface of the tank is not limited and is typically 4000 W/m² or less.

Non-limiting examples of the heating medium include water, antifreeze, a methanol water mixture (an aqueous methanol solution), gas, and steam. The heating medium may be appropriately selected in consideration of the freezing point of the compound to be purified, for example.

The crystallization tank may include a cooling mechanism and is not limited as long as it can cool the solution of a compound to precipitate crystals and can form a slurry containing the crystals and a mother liquor. Crystallization tanks including a cooling mechanism are roughly classified into a type where crystals are formed by directly cooling the inside of the tank (excluding the area with which the solution or slurry is to be brought into contact) with a cooling jacket directly attached to the tank and a type where crystals are formed by performing cooling in circulation where the tank and a cooling mechanism separated from the tank are connected with a pipe.

The type where a cooling jacket is directly attached to the tank has the advantage of reducing the number of devices, but requires a larger tank for a larger heat transfer area. When a higher productivity is required, the size of the tank is excessively large, which is disadvantageous in terms of initial investment and installation area.

In this respect, in the case where the size of the tank is limited or in the case of purification of compounds requiring high productivity, the crystallization tank is preferably of a type that the contents of the tank are cooled outside the tank. In such a case where the tank is connected to a cooling mechanism with a pipe, a portion of the solution of a compound (or crystal-containing slurry) in the tank is sent to the cooling mechanism for forming crystals in the cooling mechanism, and the slurry containing the formed crystals is returned to the tank, the heat transfer area can be easily increased by increasing the number of cooling mechanisms, and the crystallization tank can be easily scaled-up.

The cooling mechanism in this case is not limited as long as it can cool the solution of a compound to precipitate crystals. Preferred examples of the cooling mechanism include those that can have a large heat transfer area, such as a shell-and-tube heat exchanger and a spiral heat exchanger, and those that perform crystallization by scraping crystals formed on the cooled surfaces, such as a cooling disk crystallizer and a scraped surface cooling crystallizer.

The cooling disk crystallizer is not limited as long as it can cool the solution of a compound to precipitate crystals and scrape off the precipitated crystals. The cooling disk crystallizer may be, for example, one including a cylinder and cooling plates that separate the inside of the cylinder and having a structure in which crystals are formed on the wall surfaces of the cooling plates and an agitator blade with a wiper is rotated in the cylinder to scrape off the crystals.

The scraped surface cooling crystallizer is not limited as long as it can cool the solution of a compound to precipitate crystals and scrape off the precipitated crystals. The scraped surface cooling crystallizer may be, for example, one including a double pipe and having a structure in which a cooling medium passes through the outer pipe and the solution of a compound (or crystal-containing slurry) in the tank passes through the inner pipe to form crystals on the wall surface of the inner pipe, and a shaft having a scraping blade is rotated in the inner pipe to scrape off the crystals.

The crystallization temperature in the crystallization tank may be appropriately adjusted according to the type of the compound to be purified. The crystallization temperature is approximately -1°C to -15°C, preferably -1.5°C to -13.5°C, more preferably -3.5°C to - 12.5°C, still more preferably -5°C to -11.5°C from the melting point of the pure substance. When the compound to be purified is a (meth)acrylic acid, the crystallization temperature is preferably 0°C to 12°C, more preferably 1°C to 10°C, still more preferably 2°C to 8.5°C. When the temperature in the crystallization tank is high, high-purity crystals are formed, but in the case of using a scraped surface cooling crystallizer in the crystallization tank, for example, scraping off the crystals in the crystallization tank may require a large amount of power or other problems may occur. When the temperature difference between the cooling medium and the inside of the crystallization tank is too large, problems may occur such as blocking of the scraper during use of the scraped surface cooling crystallizer in the crystallization tank, for example. This may lead to a difficulty in continuous operation. Thus, when the temperature in the crystallization tank is high, the temperature difference between the cooling medium and the inside of the crystallization tank is required to be reduced, and the amount of crystals formed per unit heat transfer area is required to be reduced. When the temperature in the crystallization tank is low, low-purity crystals are formed, but in the case of using a scraped surface cooling crystallizer in the crystallization tank, the crystallization tank requires only a small amount of power for scraping off crystals. Thereby, the scraper is less likely to be blocked even when the temperature difference between the cooling medium and the inside of the crystallization tank is large. As a result, the temperature difference between the cooling medium and the inside of the crystallization tank may be increased to increase the amount of crystals formed per unit heat transfer area. However, when the crystallization temperature is too low, crystals with smaller particle sizes, which are less likely to settle, are formed.

The ripening tank is not limited as long as it can keep the crystals of a compound suspended in the tank. When the crystals are kept for a certain period of time, fine crystals are melted by Ostwald ripening and large crystals grow further, so that the crystal size distribution becomes narrow. Even in the crystallization tank, the same effect as in the ripening tank can be expected by keeping the crystals for a certain period of time in the crystallization tank.

The ripening tank typically includes, in the vicinity of the bottom, a discharge port for discharging the slurry containing crystals of a compound from the ripening tank.

The ripening tank may have a baffle in the tank.

Examples of a material for the baffle include metals such as stainless steel and resins.

The ripening tank may include multiple baffles.

Preferably, the ripening tank may further include, in the vicinity of the top roof, a discharge port for discharging the mother liquor in a supernatant part from the ripening tank. The discharged mother liquor can be recycled. Thereby, the yield of the compound can be improved. For example, the discharged mother liquor can be returned to the tank in the previous step (preceding stage). The nozzle or pipe that constitutes the discharge port may be made of any material. Examples of the material include metals and alloys.

The ripening tank may include one or more discharge ports for a mother liquor.

The ripening tank may include a mechanism for preventing the crystals of a compound from entering the discharge port for a mother liquor (e.g., a partition plate or a weir) . Such a mechanism further prevents crystals from entering the discharge port for a mother liquor.

The crystallization tank or the ripening tank may have any size. For example, each tank preferably has an inner diameter of 100 to 50000 mm. The tank preferably has a length of 1000 to 100000 mm.

The retention time of the compound in the ripening tank may be appropriately adjusted according to the type of the compound to be purified. The retention time is preferably 0.5 to 6 hours in order to adjust the particle size distribution of the slurry to be sent to the wash column and to reduce the reflux ratio (flow rate of washing liquid/flow rate of compound to be purified) in the wash column. When the compound is a (meth)acrylic acid, the retention time is more preferably 1 to 5 hours, still more preferably 1.2 to 4.5 hours.

The retention time is a value calculated by dividing the volume of the suspension part in the ripening tank by the flow rate of the slurry fed from the ripening tank to the wash column in the next step (subsequent stage).

The size of the crystallization tank is determined based on the required heat transfer area and the like. The retention time of the compound in the crystallization tank depends on the operating conditions.

Instruments such as a thermometer, a pressure gauge, a liquid level gauge (e.g., of radar type), and a level switch (e.g., of float type) may be provided in the main body or periphery of the crystallization tank or the ripening tank. These instruments may be covered with a cover. Also, the ripening tank may include a sight glass (observation window) in its side wall or the like, a hole such as a manhole or a hand hole (a hole for inserting a hand for maintenance) in its top roof, side wall, or the like, and a rupture device or the like in its top roof. The numbers of these are not limited.

The tank of the present invention is not limited to the tank in use. The tank of the present invention is not limited as long as it can have the above-mentioned structure so that during the use of the tank of the present invention, the solution or slurry can be brought into contact with the heated area of the inner wall surface of the tank.

FIG. 1 is a schematic cross-sectional side view of an exemplary tank of the present invention.

The solution or slurry 11 is fed through a nozzle 12 into the tank 1 (FIG. 1 shows a ripening tank including an agitator 3). Here, the nozzle 12 vertically penetrates the top roof of the tank 1 and has a bent tip in which the feed port is directed to and close to the heated area (the area heated by a heating mechanism 13) of the inner wall surface of the tank. In this way, when the solution or slurry 11 is fed along the inner wall surface of the tank, liquid splashing can be prevented, which will occur when the solution or slurry 11 is fed dropwise directly onto the liquid surface. Further, when the solution or slurry 11 is fed while being brought into contact with the heated area of the inner wall surface of the tank, formation of crystal nuclei on the inner wall surface can be prevented and the crystal nuclei can be prevented from scaling and growing into coarse crystals.

FIG. 2 is a perspective view of the nozzle and heating mechanism in the tank shown in FIG. 1.

In FIG. 2, the heating mechanism is a jacket-type heating mechanism and can heat an area larger in vertical and horizontal lengths than the inner diameter of the nozzle 12. Thereby, formation of crystal nuclei on the inner wall surface can be sufficiently prevented.

FIG. 3 is a schematic cross-sectional side view of an exemplary tank of the present invention.

The tank shown in FIG. 3 includes not only the nozzle 12 as shown in FIG. 1 but also a nozzle 12a. A solution or slurry 11a is fed into the tank 1 through the nozzle 12a. Here, like the nozzle 12, the nozzle 12a also vertically penetrates the top roof of the tank 1 and has a bent tip in which the feed port is directed to and close to the heated area of the inner wall surface of the tank. In this way, when the solution or slurry 11a is fed along the inner wall surface of the tank, liquid splashing can be prevented, which will occur when the solution or slurry 11a is fed dropwise directly onto the liquid surface, and when the solution or slurry 11a is fed while being brought into contact with the heated area of the inner wall surface of the tank, formation of crystal nuclei on the inner wall surface can be prevented and the crystal nuclei can be prevented from scaling and growing into coarse crystals.

When the tank includes a nozzle for feeding a slurry and a nozzle for feeding a solution, the heights of the tips of these nozzles may be the same as each other, or the height of the tip of the nozzle for feeding a slurry may be lower than the height of the tip of the nozzle for feeding a solution.

When the nozzles are arranged such that the height of the tip of the nozzle for feeding a slurry is lower than the height of the tip of the nozzle for feeding a solution, the height of the tip of the nozzle for feeding a slurry is preferably lower than the height of the tip of the nozzle for feeding a solution by 10 to 500 mm, more preferably by 50 to 400 mm, still more preferably by 80 to 300 mm. With such a structure, scattering of the slurry, adherence of crystals to the inner wall surface, and growth of the crystals as nuclei can be prevented, and even when the slurry scatters to be adhered to the inner wall surface as scales, the scales can be washed away while they are small.

Here, the height of the tip of the nozzle refers to the height of the center of the opening of the tip face of the nozzle. The difference between the heights is the difference in height in the vertical direction.

For example, preferably, a slurry is fed into the tank 1 through the nozzle 12 and a solution is fed into the tank 1 through the nozzle 12a as shown in FIG. 3.

Also, preferably, the direction in which the solution is discharged from the nozzle for feeding a solution is the same as the direction in which the slurry is discharged from the nozzle for feeding a slurry. The direction in which the solution or slurry is discharged from the nozzle refers to the direction of the extended centerline of the opening of the tip of the nozzle.

From the same point of view, the height of the position where the extended centerline of the opening of the tip of the slurry-feed nozzle intersects the heating mechanism may be the same as the height of the position where the extended centerline of the opening of the tip of the solution-feed nozzle intersects the heating mechanism. Alternatively, the height of the position where the extended centerline of the opening of the tip of the slurry-feed nozzle intersects the heating mechanism may be lower than the height of the position where the extended centerline of the opening of the tip of the solution-feed nozzle intersects the heating mechanism.

When the height of the position where the extended centerline of the opening of the tip of the slurry-feed nozzle intersects the heating mechanism is lower than the height of the position where the extended centerline of the opening of the tip of the solution-feed nozzle intersects the heating mechanism, the difference between the heights is preferably 10 to 500 mm, more preferably 50 to 400 mm, still more preferably 80 to 300 mm. Thereby, scales formed on the inner wall surface can be constantly washed away with the solution during operation of the purification apparatus.

One or more heating mechanisms may be installed for a group of multiple nozzles such as the nozzle 12 and the nozzle 12a, or one or more heating mechanisms may be installed for each nozzle.

The nozzle for feeding a slurry and the nozzle for feeding a solution are preferably located close to each other with a horizontal distance therebetween when viewed from above. The horizontal distance between the nozzles is preferably 5 to 2500 mm, more preferably 10 to 1500 mm, still more preferably 15 to 900 mm. The horizontal distance refers to the distance (distance of the horizontal component) between the centers of the nozzles viewed from above. With such a structure, scattering of the slurry, adherence of crystals to the inner wall surface, and growth of the crystals as nuclei can be prevented, and even when the slurry scatters to be adhered to the inner wall surface as scales, the scales can be washed away while they are small.

### (Purification apparatus of the present invention)

The present invention also relates to a purification apparatus including the tank of the present invention.

When the purification apparatus of the present invention includes a crystallization tank, the purification apparatus of the present invention may include one or multiple crystallization tanks. When the purification apparatus of the present invention includes multiple crystallization tanks (first to N-th tanks), these crystallization tanks are preferably connected in series. In this case, the purification apparatus of the present invention typically includes a line for sending the slurry containing crystals of a compound from one crystallization tank to another crystallization tank optionally via a solid-liquid separator. In this case, in the purification apparatus of the present invention, at least one crystallization tank includes a line for feeding a liquid to be purified containing a compound. When the purification apparatus of the present invention further includes a ripening tank, preferably, at least the N-th crystallization tank in the purification apparatus of the present invention includes a line for feeding the slurry containing crystals of a compound into the ripening tank.

When the purification apparatus of the present invention includes multiple tanks as described above, at least one tank should be the tank of the present invention. Preferably, all of the tanks are each the tank of the present invention.

The purification apparatus of the present invention is preferably one capable of performing continuous purification. For example, the purification apparatus can further include a wash column (preferably a wash column that forcibly transports crystals) as a subsequent stage of the tank of the present invention.

When the purification apparatus of the present invention further includes a wash column, the purification apparatus of the present invention preferably includes a line for feeding the slurry containing crystals of a compound from a tank in the purification apparatus of the present invention (e.g., when the purification apparatus of the present invention includes tanks connected in series, the tank arranged last in series) into the wash column.

Preferably, the purification apparatus of the present invention further includes a line for sending out the product from the wash column.

The purification apparatus of the present invention may further include a line for returning the mother liquor from a tank or unit in a subsequent stage to a tank or unit in a preceding stage.

The purification apparatus of the present invention may further include a mechanism that controls the amount of the slurry to be sent and the amount of the mother liquor to be returned. Examples of the control mechanism include valves attached to the lines.

The purification apparatus of the present invention may appropriately include different units commonly used in the purification apparatus.

### (Method for producing compound of the present invention)

The present invention also relates to a method for producing a compound, the method including: feeding a compound-containing solution or a slurry containing crystals of a compound into a tank while the solution or slurry is brought into contact with an inner wall surface of the tank; and heating an area of the inner wall surface with which the compound-containing solution or the slurry containing crystals of a compound is to be brought into contact,
the tank being at least one of a crystallization tank that forms a slurry containing crystals of a compound or a ripening tank that is capable of keeping crystals of a compound suspended therein.

In the method for producing a compound of the present invention, the feeding into the tank and the heating are essentially performed in this order on an object to be purified. The following describes the feeding into the tank and the heating in this order, followed by descriptions of other steps. In the case of continuous purification, the steps are typically performed simultaneously when the purification apparatus is viewed as a whole.

Herein, the term "compound" refers to a compound obtainable by the production method of the present invention, and does not refer to raw materials, by-products, and solvents in the production method of the present invention. The term "compound" may also be referred to as a "target compound" or a "target substance". Herein, the term "impurities" refers to components other than the "compound", such as raw materials, by-products, and solvents.

### <Feeding compound-containing solution or slurry containing crystals of compound into tank while solution or slurry is brought into contact with inner wall surface of tank>

In the feeding into the tank, the solution or slurry is fed into the tank while the solution or slurry is brought into contact with the inner wall surface of the tank. The area with which the slurry is to be brought into contact is heated in the heating, as will be described later. The slurry is a suspension of the crystals of a compound and a mother liquor. In other words, the liquid portion of the slurry containing crystals of a compound to be fed into the tank is the mother liquor. The crystal-containing slurry can be obtained by forming crystals in a compound-containing solution (e.g., a crude (meth)acrylic acid aqueous solution or a crude (meth)acrylic acid solution). The compound-containing solution may be prepared in-house or procured from outside sources. The compound-containing solution encompasses a crude compound.

In the feeding into the tank, a solution or a slurry containing crystals of a compound is preferably fed into the ripening tank from near the top roof of the ripening tank. For example, the solution or slurry is preferably fed into the ripening tank through the nozzle provided on the top roof of the ripening tank.

In the feeding into the tank, the solution or slurry can be fed into the tank while the solution or slurry is brought into contact with the inner wall surface of the tank using, for example, the above-mentioned nozzle.

In the feeding into the tank, the solution or slurry may be fed at any feed rate. For example, the feed rate is 0.2 × 10³ to 4.0 × 10⁵ kg/h for a nozzle in an industrial-scale ripening tank.

In the feeding into the tank, the feed temperature of the solution or slurry can be appropriately selected according to the melting point of the compound or the like. For example, the feed temperature may be appropriately adjusted within the range of 0°C to 80°C.

For example, when the compound is a (meth)acrylic acid, the feed temperature of the solution or slurry is preferably 5°C to 13°C, more preferably 6°C to 12°C.

The feed temperature of the solution or slurry is the temperature of the mother liquor in the solution or slurry immediately before being fed into the tank (e.g., the solution or slurry in the nozzle for feeding the solution or slurry to the ripening tank).

The solution to be fed into the tank contains the compound. Examples of the solution include the compound and an aqueous solution of the compound. The solution typically contains impurities other than the compound and water.

In the method for producing a compound of the present invention, the purity (mass percentage) of the compound in the solution to be fed into the tank is preferably 99% by mass or less.

Preferably, the mass percentage of the compound in the solution is 80% by mass or more.

In order to more stably obtain a product, the mass percentage of the crystals in the crystal-containing slurry to be fed into the tank is preferably 25% by mass or more, more preferably 30% by mass or more, still more preferably 35% by mass or more.

In order to achieve excellent fluidity of the slurry and to further reduce the risk of pipe clogging, the mass percentage of the crystals is preferably 55% by mass or less, more preferably 50% by mass or less, still more preferably 45% by mass or less.

The crystal-containing slurry to be fed into the ripening tank may be one concentrated by a solid-liquid separator, for example.

Herein, in the case of a simple "crystal-containing slurry to be fed into the tank", the "crystal-containing slurry to be fed into the tank" refers to the crystal-containing slurry immediately before being fed into the ripening tank, and, for example, refers to the crystal-containing slurry in a pipe or nozzle for feeding the crystal-containing slurry into the ripening tank.

Preferably, in the crystal-containing slurry to be fed into the tank, the mother liquor contains the compound. Examples of the mother liquor include the compound and an aqueous solution of the compound. The mother liquor typically contains impurities other than the compound and water.

In the method for producing a compound of the present invention, the purity (mass percentage) of the compound in the mother liquor in the crystal-containing slurry to be fed into the tank is preferably 99% by mass or less.

Preferably, the mass percentage of the compound in the mother liquor is 80% by mass or more.

In the production method of the present invention, the compound is preferably an easily-polymerizable compound having a reactive double bond.

In particular, in the production method of the present invention, the compound is more preferably an unsaturated carboxylic acid, still more preferably a (meth)acrylic acid, particularly preferably acrylic acid. Herein, the term "(meth)acrylic acid" refers to acrylic acid and/or methacrylic acid.

The tank may be operated with its interior being under increased pressure, atmospheric pressure, or reduced pressure.

The feeding into the tank may be intermittent, preferably essentially continuous during use of the tank.

### <Heated area of inner wall surface with which compound-containing solution or slurry containing crystals of compound is to be brought into contact>

In the heating, the area of the inner wall surface with which the solution or slurry is to be brought into contact is heated.

The heating can be suitably performed using, for example, the above-described heating mechanism (preferably, a jacket-type heating mechanism).

The heating temperature can be appropriately selected from the viewpoint of preventing crystals from adhering to the inner wall surface, and can be appropriately adjusted to be higher than the temperature of the compound-containing solution or slurry containing crystals of a compound by approximately +3°C to +100°C, preferably by +7°C to +80°C, still more preferably by +12°C to +60°C.

For example, when the compound is a (meth)acrylic acid, the heating temperature is preferably 17°C to 100°C, more preferably 20°C to 80°C, still more preferably 25°C to 60°C.

The heating temperature may be determined by measuring the area of the outer wall surface corresponding to the area with which the slurry is to be brought into contact using a thermometer or by measuring the temperature of the heating medium when the heating mechanism is a jacket-type heating mechanism.

The heating may be intermittent, preferably essentially continuous during use of the tank.

The position of the heating mechanism is typically higher than the liquid level in the tank.

### <Feeding into wash column>

The production method of the present invention may further include discharging the slurry containing crystals of a compound from a tank (e.g., when the purification apparatus of the present invention includes multiple tanks connected in series, the tank arranged last in the series), and feeding the slurry containing crystals of a compound into the wash column.

In the feeding into the wash column, first, the slurry containing crystals of a compound is discharged from the tank. The slurry containing crystals of a compound is preferably discharged from near the bottom of the tank.

The discharged slurry containing crystals of a compound is then fed into the wash column.

In the feeding into the wash column, the slurry containing crystals of a compound is preferably fed into the wash column from the top roof of the wash column or from near the top roof. For example, the slurry containing crystals of a compound is preferably fed into the wash column through the nozzle provided on the top roof of the wash column.

The feeding into the wash column can be suitably performed using a pump such as a centrifugal pump, a diaphragm pump, or a rotary pump.

The mass percentage of the crystals in the crystal-containing slurry to be fed into the wash column is preferably 1% by mass or more, more preferably 3% by mass or more, still more preferably 5% by mass or more, particularly preferably 10% by mass or more.

The mass percentage of the crystals is preferably 50% by mass or less, more preferably 40% by mass or less, still more preferably 30% by mass or less, particularly preferably 20% by mass or less.

Herein, in the case of a simple "crystal-containing slurry to be fed into the wash column", the crystal-containing slurry to be fed into the wash column refers to the crystal-containing slurry immediately before being fed into the wash column, and, for example, refers to the crystal-containing slurry in a pipe or nozzle for feeding the crystal-containing slurry into the wash column.

Like the crystal-containing slurry to be fed into the ripening tank, preferably, the crystal-containing slurry to be fed into the wash column contains the compound in its mother liquor. Examples of the mother liquor include the compound and an aqueous solution of the compound. The mother liquor typically contains impurities other than the compound and water.

The preferred ranges of the purity of the compound, the mass percentage of water, and the mass percentage of impurities other than the compound and water in the mother liquor are the same as the preferred ranges of the purity of the compound, the mass percentage of water, and the mass percentage of impurities other than the compound and water in the mother liquor in the pouring step, which is described later.

In the feeding into the wash column, the crystal-containing slurry may be fed at any feed rate. For example, the feed rate is 0.2 × 10³ to 4.0 × 10⁵ kg/h in an industrial-scale wash column.

In the feeding into the wash column, the feed temperature of the crystal-containing slurry can be appropriately selected according to the melting point of the compound or the like. For example, the feed temperature may be appropriately adjusted within the range of 0°C to 80°C.

For example, when the compound is a (meth)acrylic acid, the feed temperature of the crystal-containing slurry is preferably 5°C to 13°C, more preferably 6°C to 12°C.

The feed temperature of the crystal-containing slurry is the temperature of the mother liquor in the crystal-containing slurry immediately before being fed into the wash column (e.g., the crystal-containing slurry in the pipe or nozzle for feeding the crystal-containing slurry into the wash column).

### <Agitating in ripening tank>

The production method of the present invention may include agitating the slurry containing crystals of a compound in the ripening tank.

In the agitating, the crystal-containing slurry is agitated typically using an agitator provided in the ripening tank.

In the agitating, the rotation speed of the agitator is preferably in the range of 5 to 500 rpm, more preferably in the range of 10 to 300 rpm.

The agitating may be intermittent, preferably essentially continuous during use of the ripening tank.

### <Discharging mother liquor from ripening tank>

The production method of the present invention may include discharging a mother liquor in the supernatant part of the ripening tank.

The discharged mother liquor can be recycled and reused. The discharged mother liquor may be fed into the preceding crystallization tank for reuse, for example. Thereby, the quality of the compound can be further improved.

The mother liquor may be discharged using a pump or the like.

### <Obtaining crystal-containing slurry>

The production method of the present invention preferably further includes obtaining a slurry containing crystals of a compound from a compound-containing solution.

The compound-containing solution is preferably a crude (meth)acrylic acid aqueous solution or a crude (meth)acrylic acid solution. The crude (meth)acrylic acid aqueous solution refers to a solution in which a (meth)acrylic acid is dissolved in water and which contains impurities such as by-products produced during the production of the (meth)acrylic acid. The crude (meth)acrylic acid solution refers to a solution consisting of a (meth)acrylic acid and impurities such as by-products produced during the production of the (meth)acrylic acid. These can be obtained, for example, as follows: propylene and isobutylene are subjected to a vapor phase oxidation reaction to obtain a compound gas as a reaction product, and the compound gas is collected in an absorption column and optionally distilled. They are not limited to those synthesized in-house and may be procured from outside sources. The crude (meth)acrylic acid aqueous solution or the crude (meth)acrylic acid solution may be cooled, for example, using the above-described crystallization tank. Thereby, a slurry containing (meth)acrylic acid crystals can be obtained.

Examples of the impurities include acids such as propionic acid, acetic acid, maleic acid, benzoic acid, and acrylic acid dimers; aldehydes such as acrolein, furfural, formaldehyde, and glyoxal; acetone; and protoanemonin. In addition, solvents such as toluene and methyl isobutyl ketone may be contained.

Impurities in the compound-containing solution can be sufficiently removed by the production method of the present invention.

### <Obtaining compound-containing solution>

The production method of the present invention preferably further includes obtaining the compound-containing solution from a raw material.

The obtaining the compound-containing solution may be any process that can provide the compound-containing solution. When the compound is a (meth)acrylic acid, the obtaining the compound-containing solution can be suitably carried out by synthesizing acrylic acid, collecting the acrylic acid, and the like, as described in JP 2007-182437 A (Patent Literature 1), for example.

In the method for producing a compound of the present invention, the (meth)acrylic acid is preferably produced from at least one raw material selected from the group consisting of propane, propylene, acrolein, isobutene, methacrolein, acetic acid, lactic acid, isopropanol, 1,3-propanediol, glycerol, and 3-hydroxypropionic acid. The (meth)acrylic acid and/or the raw material may also be bio-based (meth)acrylic acids derived from renewable raw materials.

In the obtaining the compound-containing solution, impurities such as by-products are essentially formed. For example, when the compound is a (meth)acrylic acid, the impurities generated include water; acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin. Such impurities can be separated with excellent efficiency by a technique such as purification using the ripening tank in the production method of the present invention. Thereby, a product can be efficiently obtained.

### (Method for purifying compound)

The present invention also relates to a method for purifying a compound, the method including: feeding a compound-containing solution or a slurry containing crystals of a compound into a tank while the solution or slurry is brought into contact with an inner wall surface of the tank; and heating an area of the inner wall surface with which the compound-containing solution or the slurry containing crystals of a compound is to be brought into contact, the tank being at least one of a crystallization tank that forms a slurry containing crystals of a compound or a ripening tank that is capable of keeping crystals of a compound suspended therein.

The purification method of the present invention can stably purify a compound.

Preferred embodiments of the purification method of the present invention are the same as the preferred embodiments of the production method of the present invention described above.

### EXAMPLES

The present invention will be described in more detail below with reference to examples, but the present invention is not limited by the following examples, and appropriate modifications may be made within the scope that can conform to the gist of the above and later descriptions. All of them are included in the technical scope of the present invention.

Unless otherwise specified, "%" indicates "% by mass" and "parts" indicates "parts by mass."

### <Example 1>

### (Method for preparing acrylic acid aqueous solution)

An acrylic acid aqueous solution was prepared according to the method described in WO 2010/032665 as follows: propylene was catalytically oxidized in vapor phase to obtain an acrylic acid-containing gas; and the acrylic acid-containing gas was treated in an absorption column.

### (Method for preparing feed slurry)

An acrylic acid aqueous solution was fed into the crystallization tank. A cooling medium was fed to a jacket provided around the wall of the crystallization tank for indirect cooling. The resulting crystals adhered to the inner surface of the crystallization tank were scraped off with a scraper provided in the crystallization tank. Thus, a crystal-containing slurry (feed slurry) was prepared.

### (Purification apparatus/Purification conditions)

The purification apparatus used included the above-mentioned crystallization tank as a preceding stage and a ripening tank. The ripening tank includes the following equipment:
nozzles each for feeding a solution or a crystal-containing slurry while the solution or slurry is brought into contact with the inner wall surface of the tank (one solution-feed nozzle and one slurry-feed nozzle, each of which vertically penetrates the top roof of the ripening tank, has a bend in the ripening tank, and has a tip directed to the inner wall surface of the tank; the tip is directed downward at an angle of 45° with respect to the horizontal direction; each nozzle has an inner diameter of 80 mm and is located 50 mm away from the inner wall surface; the shape of the tip of the nozzle is such that the tip surface, which is constituted by the opening and the peripheral portion thereof, is parallel to the inner wall surface of the tank; and the feed temperature of the slurry is 8°C and the feed temperature of the solution is 10°C); and
a heating mechanism for heating an area of the inner wall surface with which the solution or crystal-containing slurry is to be brought into contact (the heating temperature is 32°C; the fluid to be heated is water; the heating mechanism has a vertical length of 500 mm, a horizontal length of 2600 mm, and a rectangular shape; the tip of the slurry-feed nozzle and the tip of the solution-feed nozzle are each located such that if the centerline of the opening of the tip of the nozzle is extended, the centerline reaches a region 200 mm from the bottom of the heating mechanism and a region 1300 mm from the left and right sides of the heating mechanism; the direction in which the slurry is discharged from the tip of the slurry-feed nozzle is different from the direction in which the solution is discharged from the tip of the solution-feed nozzle when viewed from above; and the jacket width is 25 mm (the width of the space where the heating medium flows)).

The ripening tank was operated, and crystals of a compound were kept suspended in the ripening tank to grow the crystals. The area of the inner wall surface with which the crystal-containing slurry was to be brought into contact was heated and the crystal-containing slurry was fed along the inner wall surface. Thereby, scattering of the slurry, adherence of crystals to the inner wall surface, and growth of the crystals as nuclei into coarse crystals can be sufficiently prevented, and a product can be stably obtained.

### <Example 2>

The ripening tank was operated, and the crystals of a compound were kept suspended in the tank to grow the crystals as in Example 1, except that in the ripening tank, the direction in which the solution was discharged from the tip of the solution-feed nozzle was aligned with the direction in which the slurry was discharged from the tip of the slurry-feed nozzle and the tip of the solution-feed nozzle was located such that if the centerline of the opening of the tip of the nozzle was extended, the centerline would reach a region 300 mm from the bottom of the heating mechanism. The effects of Example 1 were obtained, scales formed on the inner wall surface were constantly washed away with a solution during operation of the purification apparatus, enabling a reduction in occurrence of scaling. Thus, a product was successfully stably obtained.

### <Comparative Example 1>

The ripening tank was operated as in Example 1, except that no heating mechanism was used. As a result, crystal nuclei were formed on the inner wall surface of the tank, causing scaling.

### REFERENCE SIGNS LIST

1: tank
3: agitator
11, 11a: solution or slurry
12, 12a: nozzle
13: heating mechanism

## Claims

1. A tank for use in a purification apparatus, the tank being at least one of a crystallization tank that forms a slurry containing crystals of a compound or a ripening tank that is capable of keeping crystals of a compound suspended therein,
the tank comprising:
a nozzle for feeding a compound-containing solution or a slurry containing crystals of a compound into the tank while the solution or slurry is brought into contact with an inner wall surface of the tank; and
a heating mechanism for heating an area of the inner wall surface with which the compound-containing solution or the slurry containing crystals of a compound is to be brought into contact.

2. The tank according to claim 1,
wherein the heating mechanism is a jacket-type heating mechanism.

3. The tank according to claim 2,
wherein the jacket-type heating mechanism on a wall surface of the tank has a vertical length and a horizontal length, each of which is equal to or greater than an inner diameter of the nozzle.

4. A purification apparatus comprising the tank according to any one of claims 1 to 3.

5. A method for producing a compound, the method comprising:
feeding a compound-containing solution or a slurry containing crystals of a compound into a tank while the solution or slurry is brought into contact with an inner wall surface of the tank; and
heating an area of the inner wall surface with which the compound-containing solution or the slurry containing crystals of a compound is to be brought into contact,
the tank being at least one of a crystallization tank that forms a slurry containing crystals of a compound or a ripening tank that is capable of keeping crystals of a compound suspended therein.

6. The method for producing a compound according to claim 5,
wherein the compound is a (meth)acrylic acid.

7. The method for producing a compound according to claim 6,
wherein the (meth)acrylic acid is produced from at least one raw material selected from the group consisting of propane, propylene, acrolein, isobutene, methacrolein, acetic acid, lactic acid, isopropanol, 1,3-propanediol, glycerol, and 3-hydroxypropionic acid.

8. A method for purifying a compound, the method comprising:
feeding a compound-containing solution or a slurry containing crystals of a compound into a tank while the solution or slurry is brought into contact with an inner wall surface of the tank; and
heating an area of the inner wall surface with which the compound-containing solution or the slurry containing crystals of a compound is to be brought into contact,
the tank being at least one of a crystallization tank that forms a slurry containing crystals of a compound or a ripening tank that is capable of keeping crystals of a compound suspended therein.
